# EUROPEAN PATENT APPLICATION

(11) **EP 2 428 113 A2**
(43) Date of publication of application: **14.03.2012**
(21) Application number: 11178138.1
(22) Date of filing: 19.08.2011
(51) Int. Cl.: A01K 11/00, A01K 29/00, A61D 1/00

(54) **RTLS tag, RTLS reader and livestock management system using the same**

(30) Priority: 10.09.2010 KR 20100089070
(71) Applicant: LG Innotek Co., Ltd., Seoul 100-714 (KR)
(72) Inventor: Chung, Won Suk, Seoul 100-714 (KR)
(74) Representative: Dreiss

(57) **Abstract**

A livestock management system according to the embodiment includes an RTLS tag attached to a livestock to transmit analysis information of a blood gathered from the livestock and ID information dedicated for the RTLS tag, a reader transmitting the analysis information and ID information transmitted thereto from the RTLS tag to a server and transmitting a distance value with respect to the RTLS tag to the server upon a request of the server, and the server calculating a position of the livestock having the RTLS tag based on the distance value transmitted thereto from the reader and transmitting position information of the livestock and the analysis information of the blood to a client according to the position of the livestock.

## Description

### CROSS-REFERENCE TO RELATED APPLICATIONS

The present application claims priority to Korean Patent Application No. 10-2010-0089070 (filed on 10 September, 2010), which is hereby incorporated by reference in its entirety.

### BACKGROUND

Recently, as the interest for the RFID (Radio Frequency Identification) has been increased, the investment and technological development for the RFID has been increased. According to the RFID technology, electronic tags are attached to objects and information is collected, stored, processed and chased by wirelessly recognizing IDs of the electronic tags, thereby providing services, such as the position measurement of the objects, remote processing of the objects, management of the objects and information communication between the objects.

In particular, an active RFID tag equipped with a battery and a transmitter can perform the long-distance data transmission, so the active RFID tag is extensively used in various fields, such as environmental inspection, military supply, and medical treatment. Recently, an RTLS (Real Time Locating System) capable of checking the position of the object equipped with the tag in real time has been spotlighted as an application of the active RFID tag. The term "real time" signifies that the position of the object can be calculated within 30 seconds after the active RFID tag sends the blink signal including ID information thereof.

The RTLS is mainly based on RSSI (Received Signal Strength Intensity/Identification) and TDOA (Time Difference of Alive) technologies.

The RSSI technology utilizes the signal intensity of a wireless mobile device or a tag. In detail, the moving distance of the object is calculated based on power loss or path loss of the signal between the tag and the AP (Access Point).

The TDOA technology estimates the position by measuring time difference for signal transmission between the tag and the AP and converting the time difference into the distance. The TDOA technology is most extensively used in the UWB (Ultra Wide Band) and the Wi-Fi system. The TDOA technology is similar to the position determination scheme employed in present mobile communication terminals and can detect the precise position in the level of 30cm by using the UWB

In particular, the RTLS can be applied to freight containers or the transportation field for physical distribution to check the moving route of the objects in real time and is useful in terms of the security and management of physical distribution.

### SUMMARY

The embodiment provides an RTLS tag attached to the livestock in order to periodically inspect the health status of the livestock and transmit data for health status of the livestock in real time.

The embodiment provides a livestock management system, which makes RTLS communication with the RTLS tag to check the livestock having the abnormal health condition and the moving route of the livestock in real time.

Technical objects of the embodiment may not be limited to the above, and other objects of the embodiment can be apprehended to those skilled in the art based on the description of the embodiment described below.

An RTLS tag according to the embodiment includes an antenna, a wireless transceiver forming a communication channel with respect to a reader of an RTLS through the antenna, a blood-gathering unit gathering a blood of a livestock, a biosensor analyzing the gathered blood and outputting analysis information corresponding to an analysis result of the blood, a first memory storing ID information dedicated for the RTLS tag, and a controller controlling to transmit the analysis information output through the biosensor and the ID information stored in the first memory to the reader through the wireless transceiver.

An RTLS reader according to the embodiment includes an antenna, a first communication unit forming a communication channel with respect to an RTLS tag through the antenna and receiving information transmitted from the RTLS tag through the communication channel, a storage unit storing information received through a wireless transceiver, a controller measuring a distance value with respect to the RTLS tag and controlling to transmit the distance value and the information received through the first communication unit to a server, and a second communication unit transmitting the distance value and the information to the server in response to a control signal of the controller, wherein the information transmitted from the RTLS tag includes analysis information of a blood gathered from a livestock to which the RTLS tag is attached.

A livestock management system according to the embodiment an RTLS tag attached to a livestock to transmit analysis information of a blood gathered from the livestock and ID information dedicated for the RTLS tag, a reader transmitting the analysis information and ID information transmitted thereto from the RTLS tag to a server and transmitting a distance value with respect to the RTLS tag to the server upon a request of the server, and the server calculating a position of the livestock having the RTLS tag based on the distance value transmitted thereto from the reader and transmitting position information of the livestock and the analysis information of the blood to a client according to the position of the livestock.

### BRIEF DESCRIPTION OF THE DRAWINGS

FIG. 1 is a view showing a livestock management system according to the embodiment;

FIG. 2 is a block view showing the structure of an RTLS tag according to the embodiment;

FIG. 3 is a block view showing the structure of an RTLS reader according to the embodiment; and

FIGS. 4 and 5 are views showing the application example of a livestock management system according to the embodiment.

### DETAILED DESCRIPTION OF THE EMBODIMENTS

Hereinafter, embodiments will be described in detail with reference to accompanying drawings so that those skilled in the art can easily work with the embodiments. However, the embodiments may have various modifications.

However, it should be noted that the disclosure is not limited to specific embodiments, but various changes, equivalents and substitutions within the sprit and technical scope of the disclosure may be included in the disclosure.

The terms "first" and "second" can be used to refer to various components, but the components may not be limited to the above terms. The terms will be used to discriminate one component from the other component. For instance, the first component may be referred to the second component and vice versa without departing from the right of the disclosure. The term "and/or" will be used to refer to the combination of plural items or any one item of the plural items.

In addition, when a component is referred to as being "connected to" or "linked to" another component, the component may be directly connected to or linked to another component or an intervening component may be present therebetween. In contrast, if a component is referred to as being "directly connected to" or "directly linked to" another component, an intervening component may not be present therebetween.

The terms used in the specification are for the purpose of explaining specific embodiments and have no intention to limit the disclosure. Unless the context indicates otherwise, the singular expression may include the plural expression. In the following description, the term "include" or "has" will be used to refer to the feature, the number, the step, the operation, the component, the part or the combination thereof without excluding the presence or addition of one or more features, the numbers, the steps, the operations, the components, the parts or the combinations thereof.

Unless defined otherwise, the terms including technical and scientific terms used in this specification may have the meaning that can be commonly apprehended by those skilled in the art. The terms, such as the terms defined in the commonly-used dictionary, must be interpreted based on the context of the related technology and must not be interpreted ideally or excessively.

Hereinafter, exemplary embodiments will be described in more detail with reference to accompanying drawings. In the following description, the same reference numbers will be assigned to the same elements and explanation about the same elements will be omitted in order to avoid redundancy.

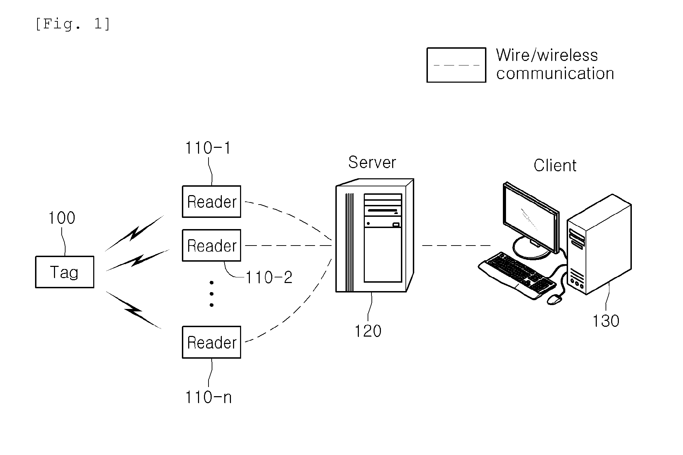

FIG. 1 is a view showing the structure of a livestock management system according to the embodiment.

Referring to FIG. 1, the livestock management system mainly includes a tag 100, at least one reader 110-1, 110-2... and 110-n, and a server 120.

The tag 100 is an active RFID tag having ID information.

The tag 100 is attached to an object (things or persons), the position of which is to be detected, and periodically transmits blink signals to the readers 110-1, 110-2... and 110-n by adding the ID information of the tag 100 to the blink signals such that the readers 110-1, 110-2... and 110-n can detect the position of the tag 100.

The readers 110-1, 110-2... and 110-n makes wireless communication with the tag 100 to control the operation of the tag 100.

In addition, the readers 110-1, 110-2... and 110-n receive information from the tag 100 to transmit the information to the server 120.

In detail, the readers 110-1, 110-2... and 110-n receive the blink singles from the tag 100 to transmit the blink signals to the server 120.

Further, the readers 110-1, 110-2... and 110-n receive the distance measurement command from the server 120. The readers 110-1, 110-2... and 110-n measure the distance value with respect to the tag 100 in response to the distance measurement command and transmit the distance value to the server 120.

The server 120 receives tag information from at least one of the readers 110-1, 110-2... and 110-n and selects at least one of the readers 110-1, 110-2... and 110-n to be used for distance measurement based on the tag information. As the reader used for distance measurement has been selected, the server 120 transmits the distance measurement command to the selected reader.

Then, the selected reader receiving the distance measurement command transmits the distance value to the server 120 so that the server 120 calculates the position of the tag 100 through the position measurement scheme based on the distance value.

More preferably, the server 120 detects the position of the object having the tag 100 based on the distance value and sends position information corresponding to the position of the object to a client 130.

The position measurement scheme may include the triangulation, which is commonly used in the field. The triangulation is a geometric scheme for estimating the position of an object moving on a two-dimensional plane in real time. In order to estimate the position of the object moving on the two-dimensional plane in real time, at least three reference points, that is, at least three readers 110-1, 110-2... and 110-n are necessary.

The client 130 requests required information to the server 120 according to the request command of a user. If the client 130 receives the required information from the server 130 according to the request command of the user, the client 130 stores or displays the required information in match with application environment.

According to the embodiment, information about the present position of the object to be detected and information about the moving route of the object are transmitted to the client 130 in real time through the livestock management system having the above structure.

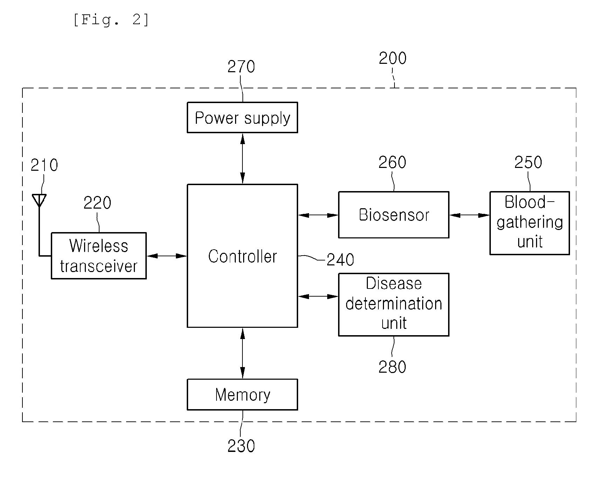

FIG. 2 is a block view showing the structure of an RTLS tag according to the embodiment.

As shown in FIG. 2, the RTLS tag 200 according to the embodiment includes an antenna 210, a wireless transceiver 220, a memory 230, a controller 240, a blood-gathering unit 250, a biosensor 260, a power supply 270 and a disease determination unit 280.

The RTLS tag 200 of the embodiment is an active RFID tag. Different from a passive tag, the active RFID tag is provided therein with the power supply 270 for supplying power to the tag. The power supply 270 may include a battery.

The RTLS tag 200 is attached to the livestock in use. However, the embodiment is not limited thereto. For instance, the RTLS tag 200 may be attached to things, such as books, or persons to be inspected other than the livestock.

The wireless transceiver 220 forms a communication channel with respect to a reader by using the antenna 210 of the RTLS and transmits/receives the signal to/from the reader through the communication channel.

The blood-gathering unit 250 periodically gathers the blood of the livestock having the RTLS tag 200 in order to inspect the blood of the livestock. The blood-gathering unit 250 includes a needle stuck into a blood vessel of the livestock. Thus, the blood of the livestock is periodically gathered in a predetermined interval through the needle.

Preferably, the needle of the blood-gathering unit 250 is consecutively stuck into the blood vessel of the livestock to periodically gather the blood from the blood vessel in a predetermined interval. If the blood has been gathered, the blood-gathering unit 250 delivers the gathered blood to the biosensor 260.

Upon receiving the gathered blood from the blood-gathering unit 250, the biosensor 260 analyzes the blood and transmits information about the blood to the controller 240.

At this time, the biosensor 260 measures components of the blood or detects the thermal variation or chemical variation of the blood and outputs analysis information including at least one of the measured components and detected variation information as an electric signal.

The blood is body fluids that circulate through the blood vessel of the person or the animal. In detail, the blood carries oxygen received from the lungs to tissue cells or discharges CO₂ from the tissue cells to the lungs. In addition, the blood carries nutrition absorbed in the digestive duct to the liver or the tissue cells. Further, the blood carries degradation products of the tissues unnecessary for a living body to the kidneys to discharge the degradation products out of the living body. In addition, the blood carries the hormone, which is secreted from the endocrine gland, to the target organ or each tissue cell or uniformly dissipates the body heat to keep the living body with the constant temperature.

Therefore, the blood can be variously utilized for the purpose of diagnose and treatment of disease. For instance, the peripheral blood inspection is to inspect the formed elements (blood-corpuscle) in the blood by observing the hematocyte, hemoglobin, hematocrit, leucocyte, and platelet. In addition, the blood biochemical inspection is to measure the chemical components contained in the blood plasma or serum, such as proteins, carbohydrates, fat, vitamins, enzymes, and inorganic substances. Further, the immunoserology is to measure the immunoglobulin (antibody) or the function of the lymphocyte using the antigen-antibody reaction.

In addition to the inspection for the components contained in the blood, the existence of disease in the livestock can be diagnosed by detecting the chemical variation or the thermal variation of the blood gathered from the livestock.

The biosensor 260 can be classified into several groups as follows according to the blood analysis schemes.

The first group of the biosensor 260 has the function of converting the chemical variation of the blood into the electric signal and may include an enzyme electrode, a hydrogen dioxide electrode and an FET (field effect transistor) electrode.

The second group of the biosensor 260 has the function of converting the thermal variation of the blood into the electric signal and may include a thermistor. In addition, the biosensor 260 may include a photo-counter/photo-diode for converting the chemical emission into the electric signal using an enzyme and an ion sensor for measuring the ion density in the electrolyte of the blood.

Thus, the biosensor 260 may include at least one of the enzyme electrode, the hydrogen dioxide electrode, the FET electrode, the thermistor, the photo-counter/photo-diode and the ion sensor.

The memory 230 stores the ID dedicated for the tag.

In addition, the memory 230 stores data for determining the existence of disease in the livestock. In detail, blood analysis data for each type of the diseases of the livestock are stored in the memory. The blood analysis data may include data of components contained in the blood or data obtained by detecting the chemical or thermal variation of the blood. For instance, if the livestock contracts a first disease, the blood analysis data may be the data of components contained in the blood of the livestock subject to the first disease or the data obtained by detecting the chemical or thermal variation of the blood of the livestock subject to the first disease.

The controller 240 receives the output signal of the biosensor 260 and converts the output signal of the biosensor 260 into digital data. The output signal is analysis information obtained by analyzing the blood of the livestock. The controller 240 controls the wireless transceiver 220 to transmit the blink signal, which is periodically transmitted in a predetermined interval, to the reader of the RTLS by adding the ID information of the tag and analysis information stored in the memory 230 to the blink signal.

In detail, the wireless transceiver 220 periodically transmits the ID information of the tag and analysis information to the reader in a predetermined interval in response to the control signal of the controller 240.

In addition, the RTLS tag 200 may further include the disease determination unit 280. The disease determination unit 280 determines whether the livestock having the RTLS tag 200 contracts the disease by using the analysis information. At this time, the disease determination unit 280 determines the disease of the livestock by using the blood analysis data stored in the memory 230. Although it has been described in that the blood analysis data are stored in the separate memory, the blood analysis data can be stored in the internal memory of the disease determination unit 280.

The controller 240 transmits the analysis information to the disease determination unit 280. More preferably, the controller 240 transmits the converted digital data to the disease determination unit 280.

The disease determination unit 280 compares the analysis information transmitted from the controller 240 with the blood analysis data stored therein to determine whether the livestock having the RTLS tag 200 contracts the disease. If the livestock contracts the disease, the disease determination unit 280 determines the type of disease of the livestock.

In this case, the controller 240 transmits information about the type of the disease of the livestock to the reader by adding the disease information to the blink signal. At this time, the controller 240 may transmit only the disease information instead of the analysis information to the reader.

The RTLS tag 200 of the embodiment is preferably employed in the stock management system, which determines whether the livestock contracts the disease in real time and trace/detect the position of the livestock when the livestock contracts the disease.

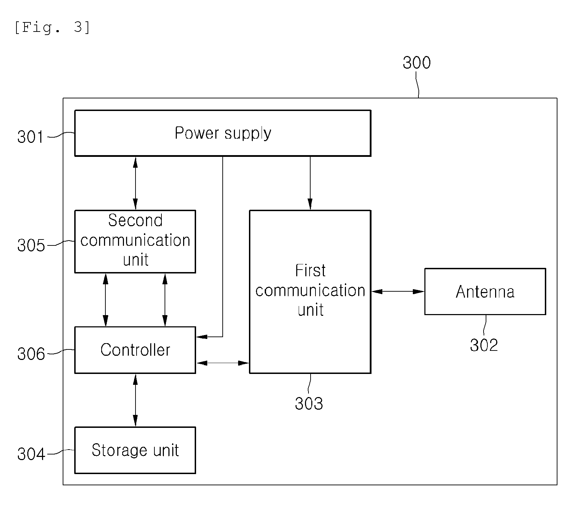

FIG. 3 is a block view showing the structure of an RTLS reader according to the embodiment.

Referring to FIG. 3, the RTLS reader 300 includes a power supply 201, an antenna 302, a first communication unit 303, a storage unit 304, a second communication unit 305 and a controller 306.

The power supply 301 supplies driving power for operating the RTLS reader 300.

The antenna 302 forms the communication channel with respect to the RTLS tag 200 to receive information from the RTLS tag 200.

The first communication unit 300 receives the information transmitted from the RTLS tag 200 through the communication channel formed by the antenna 302.

The storage unit 304 stores the program for operating the RTLS reader 300 and information generated during the operation of the RTLS reader 300.

In particular, the storage unit 304 stores the information transmitted from the RTLS tag 200.

The second communication unit 305 makes data communication with the server. In particular, the second communication unit 305 receives the distance measurement command from the server. In addition, the second communication unit 305 transmits the distance value measured according to the distance measurement command and the information stored in the storage unit 304 to the server.

The controller 306 controls the overall operation of the RTLS reader 300.

In particular, the controller 306 measures the distance value with respect to the RTLS tag 200 and performs the control operation such that the measured distance value and the information received through the first communication unit 303 can be transmitted to the server.

The information transmitted from the RTLS tag 200 may include the analysis information of the blood gathered from the livestock having the RTLS tag 200 and the ID information dedicated for the RTLS tag 200.

In addition, as described above, the analysis information of the blood may be the data for the components contained in the blood gathered from the livestock or the data obtained by detecting the thermal and chemical variation of the blood.

In addition, the controller 306 determines whether the livestock having the RTLS tag 200 contacts the disease by using the analysis information transmitted from the RTLS tag 200 and performs the control operation to transmit the determined disease information to the server.

To this end, the storage unit 304 stores blood analysis data for each type of the disease of the livestock and the controller 306 performs the control operation by using the blood analysis data stored in the storage unit 304 such that the existence of the disease in the livestock and the disease information including the type of the disease of the livestock can be transmitted to the server when the livestock contracts the disease.

At this time, the controller 306 stores the position value of the RTLS tag 200 in the storage unit 304 together with the information transmitted from the RTLS tag 200.

In addition, when the livestock having the RTLS tag 200 contracts the disease, the controller 306 transmits information about the past and present movement route of the livestock to the server by using the information stored in the storage unit 304.

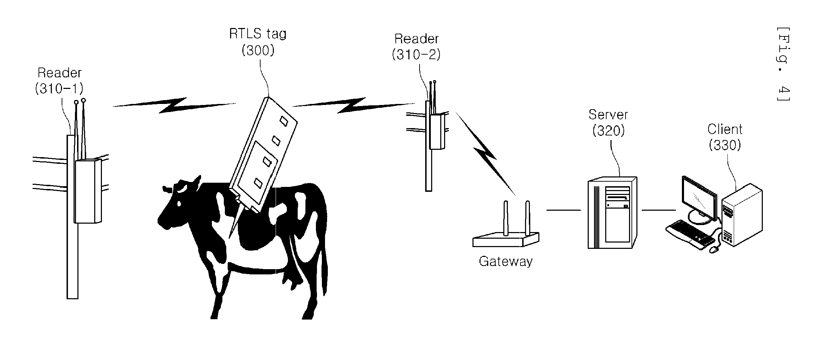

FIG. 4 is a view showing the application example of the livestock management system according to the embodiment.

FIG. 4 shows the RTLS-based livestock management system including the RTLS tag 300, a plurality of readers 310-1 and 310-2 and a server 320.

The RTLS tag 300 is attached to the livestock to analyze the blood of the livestock by periodically gathering the blood in a predetermined interval. The RTLS tag 300 transmits the analysis information and the ID information of the tag to the reader by adding the analysis information and the ID information of the tag to the blink signal.

The analysis information transmitted from the RTLS tag 300 may include data of components contained in the blood of the livestock or data obtained by detecting the chemical or thermal variation of the blood.

The readers 310-1 and 310-2 receive the blink signal of the RTLS tag 300 to transmit the blink signal to the server 320. Upon receiving the distance measurement command from the server 320, the readers 310-1 and 310-2 measure the distance value with respect to the RTLS tag 300 and transmit the distance value to the server 320.

The server 320 identifies the livestock having the RTLS tag 300 based on the ID information of the tag and calculates the position of the livestock based on the distance value with respect to the RTLS tag 300 measured by the reader.

In addition, the server 320 stores the blood analysis data for each type of the diseases of the livestock in the internal memory. The blood analysis data may include data of components contained in the blood or data obtained by detecting the chemical or thermal variation of the blood occurring when the livestock contracts the disease.

The server 320 compares the blood analysis information transmitted from the readers 310-1 and 310-2 with the blood analysis data stored in the internal memory to determine the existence of disease in the livestock and the type of the disease if the livestock contracts the disease. In addition, the server 320 transmits the determination result to a client 330.

The client 330 may detect whether there is the livestock that contracts the disease based on the information transmitted from the server 320. If there is the livestock that contracts the disease, the client can confirm the type of the disease contracted to the livestock. In addition, the client 330 can monitor the present position of the livestock that contracts the disease.

In addition, the RTLS-based livestock management system according to the embodiment operates as follows.

The RTLS tag 300 is attached to the livestock to periodically gather the blood of the livestock in a predetermined interval. The RTLS tag 300 analyzes the gathered blood and determines whether the livestock contracts the disease and the type of the disease if the livestock contacts the disease. The RTLS tag 300 transmits the determination result to the readers 310-1 and 310-2 by adding the determination result to the blink signal together with the ID information of the tag.

Upon receiving the blink signal from the RTLS tag 300, the readers 310-1 and 310-2 transmit the blink signal to the server 320. If the distance measurement command is transmitted to the readers 310-1 and 310-2 from the server 320, the readers 310-1 and 310-2 measure the distance value with respect to the RTLS tag 300 and transmit the distance value to the server 320.

The server 320 identifies the livestock having the RTLS tag 300 based on the ID information of the tag and calculates the position of the livestock by using the distance value with respect to the RTLS tag 300 measured by the readers 310-1 and 310-2.

In addition, the server 320 transmits the determination result of the disease of the livestock and the type of the disease, which are transmitted from the readers 310-1 and 310-2, together with the present position information of the livestock to the client 330 in real time.

As described above, according to the livestock management system of the embodiment, the RTLS tag 300 can determine the existence of disease in the livestock having the RTLS tag 30 and the type of the disease if the livestock contracts the disease and can transmit the determination result to the server 320 in real time. According to another embodiment, the RTLS tag 300 can transmit only the data obtained by analyzing the blood of the livestock having the RTLS tag 300 to the server 320 in such a manner that the server 320 can determine the existence of disease in the livestock and the type of the disease.

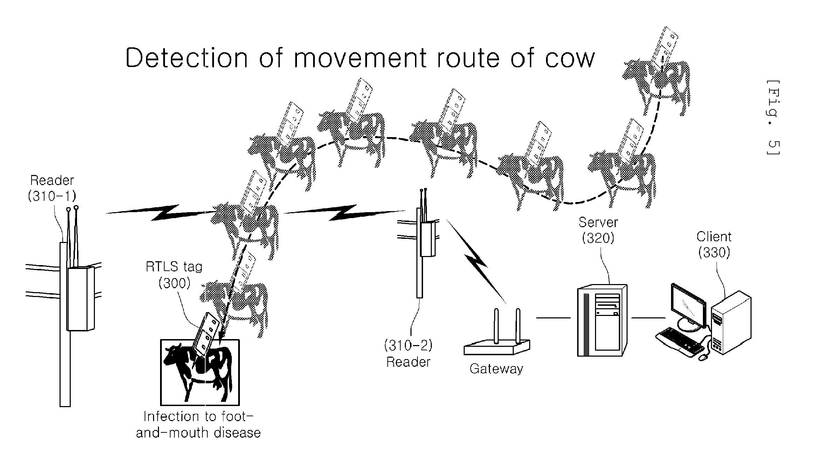

FIG. 5 is a view showing an application example of the livestock management system according to the embodiment. As shown in FIG. 5, the livestock management system according to the embodiment can transmit the information about the existence of the disease in the livestock and the type of disease to the client in real time and can collect the position information of the livestock to supply information about the past and present movement route of the livestock to the client.

In detail, the server 320 stores information about the existence of the disease in the livestock, the type of disease and the present position of the livestock and manages the stored information. In addition, the server stores and manages information about the position of the livestock having no disease.

Therefore, if the livestock having no disease contracts the disease later, the server 320 checks the past movement route of the livestock by using the previously stored information, and transmits the information about the past movement route and the present position of the livestock to the client.

The information about the past and present movement route of the livestock having the disease may be very useful data for estimating the attack point of the disease fatal to the livestock. In addition, this information may serve as an important clue for estimating the propagation route of the disease so that the propagation of the disease can be effectively prevented.

As described above, according to the embodiment, the status of the livestock can be detected in real time, so that the livestock having the disease can be found within a short period of time to treat the disease.

In addition, since the past and present movement route of the livestock having the disease can be detected, the attack point of the disease fatal to the livestock can be estimated. Thus, the propagation route of the disease can be previously estimated, so that the propagation of the disease to other livestock can be effectively prevented.

Although embodiments have been described with reference to a number of illustrative embodiments thereof, it should be understood that numerous other modifications and embodiments can be devised by those skilled in the art that will fall within the spirit and scope of the principles of this disclosure. More particularly, various variations and modifications are possible in the component parts and/or arrangements of the subject combination arrangement within the scope of the disclosure, the drawings and the appended claims. In addition to variations and modifications in the component parts and/or arrangements, alternative uses will also be apparent to those skilled in the art.

## Claims

1. An RTLS (real time locating system) tag comprising:
an antenna;
a wireless transceiver forming a communication channel with respect to a reader of an RTLS through the antenna;
a blood-gathering unit gathering a blood of a livestock;
a biosensor analyzing the gathered blood and outputting analysis information corresponding to an analysis result of the blood;
a first memory storing ID information dedicated for the RTLS tag; and
a controller controlling to transmit the analysis information output through the biosensor and the ID information stored in the first memory to the reader through the wireless transceiver.

2. The RTLS tag of claim 1, wherein the blood-gathering unit includes a needle stuck into a blood vessel of the livestock and the blood of the livestock is periodically gathered in a predetermined interval through the needle to deliver the blood to the biosensor.

3. The RTLS tag of claim 1, wherein the biosensor includes at least one of an enzyme electrode, a hydrogen dioxide electrode, a field effect transistor electrode, a thermistor, a photo counter, a photodiode and an ion sensor to detect components contained in the gathered blood or thermal or chemical variation of the blood.

4. The RTLS tag of claim 1, further comprising a second memory to store blood analysis data for each type of diseases to be contracted to the livestock, wherein the blood analysis data include components contained in the blood or data obtained by detecting chemical or thermal variation of the blood.

5. The RTLS tag of claim 4, further comprising a disease determination unit determining a type of diseases contracted to the livestock by using the data stored in the second memory, wherein the controller transmits the analysis information of the biosensor to the disease determination unit and periodically transmits the determination result of the disease determination unit and the ID information of the RTLS tag to the reader in a predetermined interval.

6. An RTLS reader comprising:
an antenna;
a first communication unit forming a communication channel with respect to an RTLS tag through the antenna and receiving information transmitted from the RTLS tag through the communication channel;
a storage unit storing information received through a wireless transceiver;
a controller measuring a distance value with respect to the RTLS tag and controlling to transmit the distance value and the information received through the first communication unit to a server; and
a second communication unit transmitting the distance value and the information to the server in response to a control signal of the controller,
wherein the information transmitted from the RTLS tag includes analysis information of a blood gathered from a livestock to which the RTLS tag is attached.

7. The RTLS reader of claim 6, wherein the blood analysis information includes measurement data of components contained in the blood gathered from the livestock or data obtained by detecting thermal or chemical variation of the blood.

8. The RTLS reader of claim 6, wherein the controller determines existence of the disease in the livestock having the RTLS tag by using the analysis information transmitted thereto, and controls to transmit disease information including the determination result for the existence of the disease in the livestock to the server.

9. The RTLS reader of claim 7, wherein the storage unit stores blood analysis data for each type of diseases contracted to the livestock and the controller controls to transmit disease information including a type of the disease contracted to the livestock to the server when the livestock contracts the disease.

10. The RTLS reader of claim 8, wherein the controller transmits information about a past and present movement route of the livestock to the server by using the information stored in the storage unit when the livestock contracts the disease.

11. A livestock management system comprising:
an RTLS tag attached to a livestock to transmit analysis information of a blood gathered from the livestock and ID information dedicated for the RTLS tag;
a reader transmitting the analysis information and ID information transmitted thereto from the RTLS tag to a server and transmitting a distance value with respect to the RTLS tag to the server upon a request of the server; and
the server calculating a position of the livestock having the RTLS tag based on the distance value transmitted thereto from the reader and transmitting position information of the livestock and the analysis information of the blood to a client according to the position of the livestock.

12. The livestock management system of claim 11, wherein the server determines existence of the disease in the livestock by using the analysis information of the blood and transmits the position information and the disease information of the livestock to the client when the livestock contracts the disease.

13. The livestock management system of claim 11, wherein the server stores blood analysis data for each type of the diseases to be contracted to the livestock and the disease information includes a type of the disease contracted to the livestock.

14. The livestock management system of claim 11, wherein the RTLS tag stores blood analysis data for each type of the diseases to be contracted to the livestock and the analysis information includes a type of the disease according to existence of the livestock.

15. The livestock management system of claim 11, wherein the blood analysis information includes measurement data of components contained in the blood gathered from the livestock or data obtained by detecting thermal or chemical variation of the blood.

16. The livestock management system of claim 11, wherein the blood analysis information and the ID information of the RTLS tag are carried by a blink signal.

17. The livestock management system of claim 11, wherein the server stores position information of the livestock received therein, and transmits information about a past and present movement route of the livestock to the client by using the stored position information.
